(19) Europäisches Patentamt
European Patent Office
Office européen des brevets

(11) EP 1 498 107 A2

(12) **DEMANDE DE BREVET EUROPEEN**

(43) Date de publication:
**19.01.2005 Bulletin 2005/03**

(51) Int Cl.[7]: **A61K 7/06**, A61K 7/13

(21) Numéro de dépôt: **04291776.5**

(22) Date de dépôt: **12.07.2004**

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL HR LT LV MK**

(30) Priorité: **16.07.2003 FR 0308677**

(71) Demandeur: **L'OREAL**
**75008 Paris (FR)**

(72) Inventeurs:
- **Rollat-Corvol, Isabelle**
  **75017 Paris (FR)**
- **Samain, Henri**
  **91570 Bievres (FR)**

(74) Mandataire: **Wattremez, Catherine**
**L'OREAL - D.I.P.I.**
**25-29 Quai Aulagnier**
**92600 Asnières (FR)**

(54) **Composition comprenant au moins un polymère conducteur et au moins un colorant direct et procédé la mettant en oeuvre**

(57) L'invention concerne une composition comprenant, dans un milieu cosmétiquement acceptable, (a) au moins un colorant direct, (b) au moins un polymère conducteur de préférence comprenant au moins une unité répétitive de type des anilines, pyrroles, thiophènes ou bisthiophènes, furanes, para-phénylène-sulfures, para-phénylène vinylènes, indoles, amides aromatiques, hydrazides aromatiques, azométhines aromatiques, esters aromatiques particuliers.
Elle concerne de plus un procédé mettant en oeuvre une telle composition, ainsi que son utilisation pour conférer un effet optique aux fibres kératiniques.

EP 1 498 107 A2

## Description

L'invention concerne une composition comprenant, dans un milieu cosmétiquement acceptable, au moins un polymère conducteur soluble et au moins un colorant direct. Elle concerne également un procédé de traitement de fibres kératiniques mettant en oeuvre la composition précitée. Elle a enfin pour objet l'utilisation d'une telle composition afin d'apporter un effet optique aux fibres kératiniques.

La présente invention a trait au domaine de la coloration capillaire et plus particulièrement à des colorations semi-permanentes obtenues au moyen de colorants directs.

Les colorants directs sont des colorées et colorantes ayant une certaine affinité avec les fibres kératiniques. Généralement, ces colorants directs sont choisis parmi les colorants benzéniques nitrés, les colorants azoïques, azométhiniques, méthiniques, anthraquinoniques, naphtoquinoniques, benzoquinoniques, phénotiaziniques indigoïdes, xanthéniques, phénanthridiniques, phtalocyanines, ceux dérivés du triarylméthane, les colorants naturels (henné par exemple), seuls ou en mélanges.

Il n'est pas rare, dans le but d'améliorer les effets optiques apportés aux fibres kératiniques après le procédé de coloration, comme des effets de brillance par exemple, d'incorporer dans la composition, des agents particuliers.
Par exemple, pour donner de la brillance aux fibres, on utilise notamment des substances hydrophobes lubrifiantes, telle que des huiles ou des cires organiques ou des silicones. Toutefois, l'effet de brillance obtenu manque d'intensité et leur donne en général un aspect artificiel.
En outre, de telles compositions présentent l'inconvénient d'apporter un toucher gras ou collant aux fibres.

Enfin, la présence de composés de ce type peut limiter la montée du colorant dans les fibres et par conséquent donner des colorations moins intenses.

La présente invention a donc pour but de proposer des compositions comprenant au moins un colorant direct, qui apportent aux fibres kératiniques traitées un aspect brillant sans les inconvénients rencontrés avec les compositions classiques, tout en conservant de bonnes propriétés de coloration, comme des couleurs tenaces, intenses, peu sélectives.

Enfin, les fibres présentent de manière avantageuse un toucher doux et agréable.

La présente invention a ainsi pour premier objet une composition comprenant, dans un milieu cosmétiquement acceptable,

(a) au moins un colorant direct,
(b) au moins un polymère conducteur.

L'invention concerne de plus un procédé de traitement des fibres kératiniques humaines, et plus particulièrement des cheveux, avec une composition comprenant lesdits polymères conducteurs.
Selon une première variante, le procédé consiste à appliquer sur les fibres sèches ou humides, une composition telle que définie précédemment, puis on évapore ou on laisse évaporer le milieu de la composition entre 20 et 120°C, de préférence entre 20 et 80°C, jusqu'à ce que les fibres soient sèches.
Selon une autre variante du procédé selon l'invention, on applique sur les fibres sèches ou humides, la composition selon l'invention. Puis on rince éventuellement les fibres, on lave éventuellement et on rince les fibres, puis on sèche ou on laisse sécher lesdites fibres entre 20 et 120°C, de préférence entre 20 et 80°C.

L'invention a de même pour objet l'utilisation d'une composition comprenant au moins un agent oxydant et au moins un polymère conducteur pour conférer un effet optique aux fibres kératiniques.

En effet, la composition selon l'invention apporte à l'ensemble de la chevelure, et de façon uniforme, notamment une brillance substantiellement plus intense, plus naturelle, et plus esthétique qu'avec les moyens de l'art antérieur.

Par ailleurs, lorsque les polymères conducteurs présents dans la composition selon l'invention absorbent dans le spectre visible, on obtient simultanément un effet optique, comme de la brillance, et de la couleur.
Cela peut permettre d'élargir la palette des couleurs susceptibles d'être obtenues, ou bien encore d'optimiser les teneurs en colorants directs.

Mais d'autres caractéristiques et avantages de l'invention apparaîtront plus clairement à la lecture de la description et de l'exemple qui suivent.

Dans ce qui va suivre et à moins d'une indication différente, les bornes d'un domaine de valeurs sont comprises comme faisant partie de ce domaine.

Au sens de la présente invention, le terme effet optique recouvre des effets de brillance, de couleur, métallique, goniochromatique, moiré.

Par ailleurs, et plus particulièrement, il est à noter que la brillance correspond à l'intensité lumineuse réfléchie sous un angle $\alpha$ lorsque la mèche de cheveux est éclairée sous un angle $-\alpha$. L'angle $\alpha$ classiquement utilisé pour mesurer cette réflexion spéculaire, autrement dit la brillance, est égal à 20°. Cet apport de brillance peut être mesuré

par utilisation d'un brillancemètre comme il est par exemple décrit dans la norme ISO 2813 - 1994 de l' AFNOR (août 1994, rectificatif février 1997).

Polymères conducteurs

Selon la présente invention, on entend par "polymère conducteur" une structure moléculaire dans laquelle le ou les monomères présentent une forte délocalisation électronique et dont la disposition dans le squelette du polymère permet aux orbitales $\pi$ de se recouvrir. Cette caractéristique chimique se traduit par un phénomène de conduction électrique qui s'accompagne ou non d'un phénomène d'absorption dans le spectre UV-visible, voire dans l'infrarouge.

Par polymère conducteur absorbant dans le visible, on entend au sens de la présente invention, tout polymère conducteur présentant une absorbance non nulle dans le domaine de longueur d'ondes allant de 400 à 800 nm, même si les maxima d'absorption du polymère se situent en dehors de cette gamme.

Les polymères conducteurs mis en oeuvre dans le cadre de la présente invention sont des polymères conducteurs solubles ou dispersibles dans le milieu cosmétique approprié à l'application.
On dit que le polymère est soluble dans le milieu lorsqu'il forme un liquide limpide isotrope à 25°C dans le milieu comprenant de l'eau ou un mélange eau/solvant ; ceci étant obtenu dans tout ou partie d'une gamme de concentration comprise entre 0,01 et 50 % en poids de polymère conducteur.
De façon préférée, les polymères conducteurs mis en oeuvre dans le cadre de la présente invention sont des polymères conducteurs solubles ou dispersibles dans un milieu aqueux, avantageusement dans l'eau.

On dit que le polymère est dispersible dans le milieu comprenant de l'eau ou un mélange eau/solvant si, à 0,01% en poids, à 25°C, il forme une suspension stable de fines particules, généralement sphériques. La taille moyenne des particules constituant ladite dispersion est inférieure à 1 µm et, plus généralement, varie entre 5 et 400 nm, de préférence de 10 à 250 nm. Ces tailles de particules sont mesurées par diffusion de lumière.
Il est à noter que de manière avantageuse, ces polymères ne nécessitent pas l'emploi d'un agent dispersant.

De préférence, les polymères conducteurs se présentent sous une forme soluble dans le milieu de la composition.

De plus, les polymères présentent de manière avantageuse, une conductivité comprise entre $10^{-5}$ et $5.10^{5}$ siemens/cm, plus particulièrement comprise entre $10^{-3}$ et $10^{5}$ siemens/cm, et de préférence comprise entre $10^{-1}$ et $10^{4}$ siemens/cm.
La conductivité est mesurée à l'aide d'un générateur de courant (RM2 Test Unit commercialisé par la société Jandel) muni d'une tête de mesure dite quatre pointes (Universal four-point probes commercialisé par la société Jandel). Les quatre pointes alignées et distantes du même espacement d sont appliquées par simple pression sur l'échantillon à analyser. Un courant I est injecté par les pointes externes à l'aide de la source de courant créant ainsi une variation de potentiel. La tension U est mesurée entre les deux pointes internes reliées au voltmètre du générateur de courant.

Dans cette configuration la conductivité de l'échantillon exprimée en S/cm est donnée par l'expression suivante :

$$\sigma = (K \times I)/(U \times e)$$

Avec :

- K coefficient dépendant de la position des contacts sur la surface de l'échantillon.
  Lorsque les pointes sont alignées et équidistantes, K est égal à : $\Pi/\log(2)$
- I : valeur du courant injecté exprimé en ampères
- U : valeur de la tension mesurée exprimée en volts
- e : épaisseur de l'échantillon exprimée en cm

Cette expression ne peut être utilisée que lorsque l'épaisseur du matériau est négligeable devant la distance d existant entre deux pointes (e/d < 0,25). Pour obtenir des épaisseurs suffisamment faibles et ainsi pouvoir calculer la conductivité du matériau, il est préconisé de réaliser la mesure sur un support non conducteur (par exemple, une lame de verre) recouvert du matériau à analyser obtenu par évaporation d'une solution diluée. Afin d'améliorer l'homogénéité du revêtement à analyser, il est également préconisé d'utiliser la technique de dépôt dite du spin coating.

Selon un mode de réalisation particulier, les polymères conducteurs présents dans la composition sont choisis parmi les polymères comprenant au moins une unité répétitive de formules suivantes :

les anilines de structure (1) suivante :

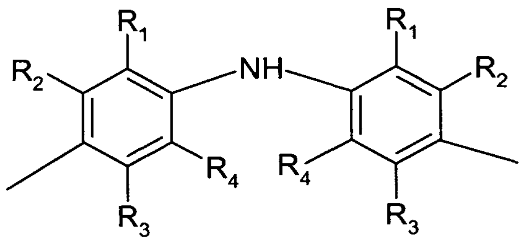

(I)

<u>les pyrroles de structure (IIa) et (IIb) suivantes</u> :

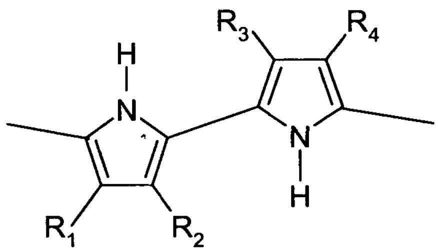

(IIa)

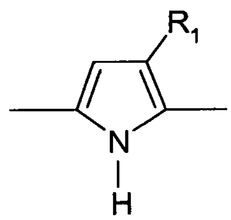

(IIb)

<u>les thiophènes ou bisthiophènes de formules (IIIa), (IIIb) et (IIIc) suivantes</u> :

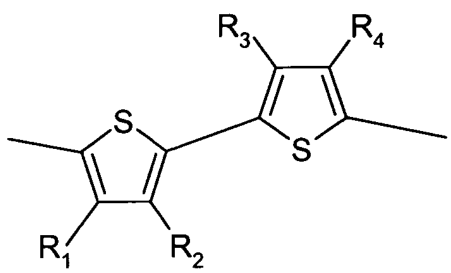

(IIIa)

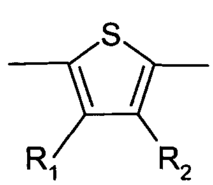

(IIIb)

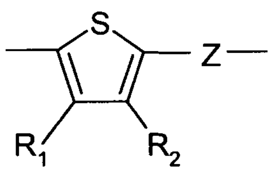

(IIIc)

les furanes de formule (IV) suivante :

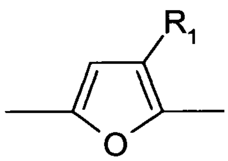

(IV)

les para-phénylène-sulfure de structure (V) suivante :

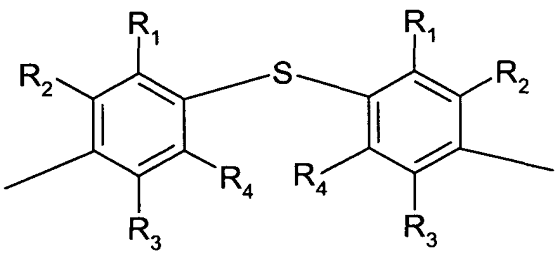

(V)

les paraphénylène vinylène de formule (VI) suivante :

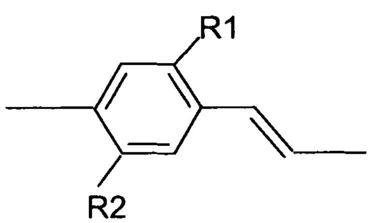

(VI)

les indoles de formule (VII) suivante :

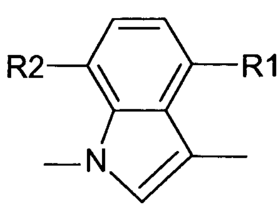

(VII)

les amides aromatiaues de formules suivantes (VIIIa), (VIIIb). (VIIIc). (VIIId) :

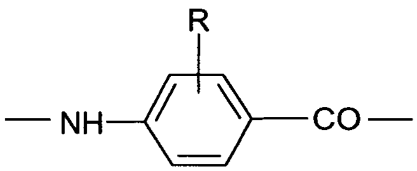

(VIIIa)

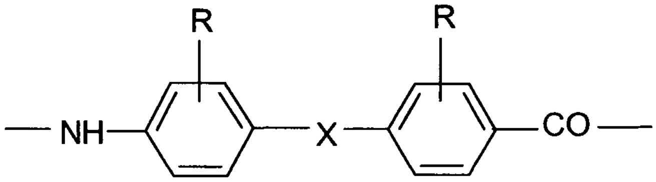

(VIIIb)

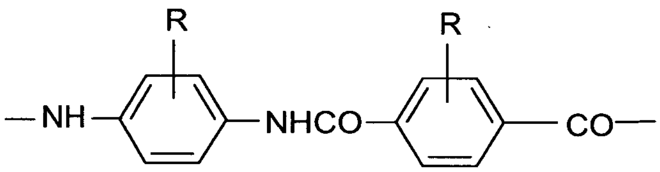

(VIIIc)

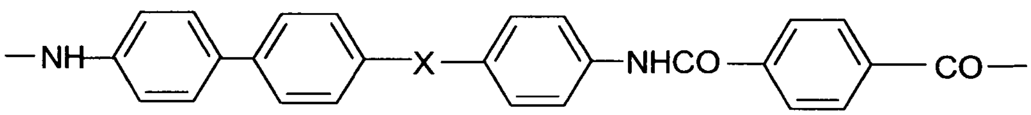

(VIIId)

les hydrazides aromatiques de formules (IXa), (IXb) et (IXc) suivantes :

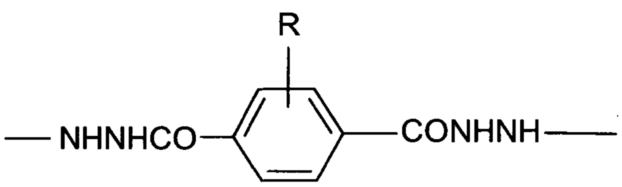

(IXa)

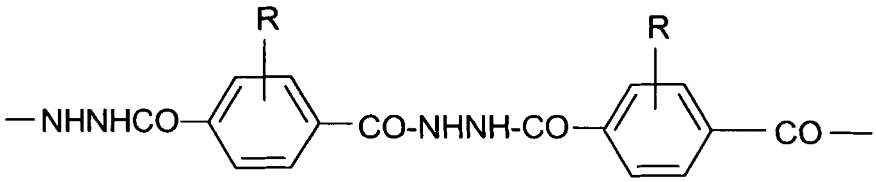

(IXb)

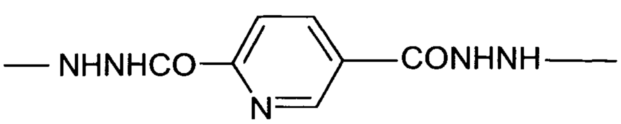

(IXc)

les azométhines aromatiques de formules (Xa), (Xb) et (Xc) suivantes :

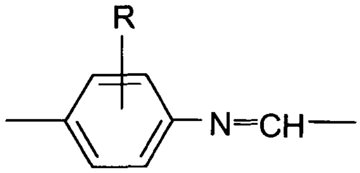

(Xa)

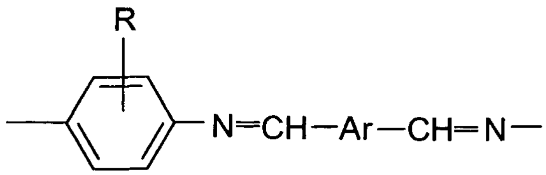

(Xb)

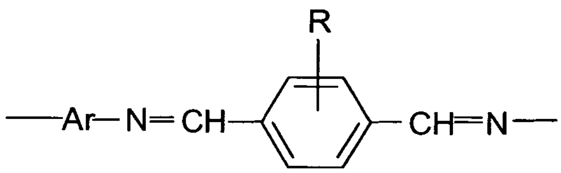

(Xc)

les esters aromatiques de formules (XIa), (XIb) et (XIc) suivantes :

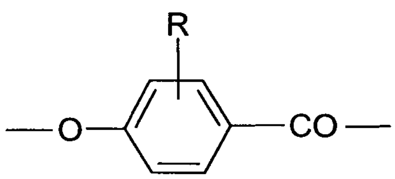

(XIa)

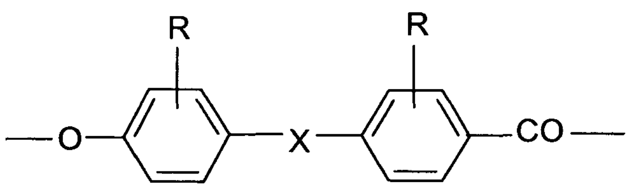

(XIb)

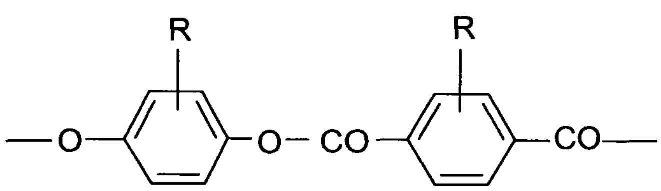

(XIc)

formules (I) à (XI) dans lesquelles :

les radicaux R, $R_1$ à $R_4$ identiques ou différents, sont choisis dans le groupe formé par l'hydrogène, un radical -R', -OR', -COOR', -OCOR', avec R' représentant un radical alkyle linéaire ou ramifié en $C_1$-$C_{20}$, un atome d'halogène, un radical nitro, un radical cyano, un radical cyanoalkyle, et un groupement solubilisant ;
Ar représente un radical comprenant un radical monoaromatique ou polyaromatique X = -NHCO-, -O-, -S-, $-SO_2-$, -N=N-, $-C(CH_3)_2-$, $-CH_2-$, -CH=CH-, -CH=N- ;

Z = -CH=CH- ou -C≡C-.

Plus particulièrement, Ar représente au moins un radical choisi parmi les suivants :

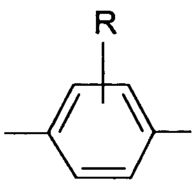 , 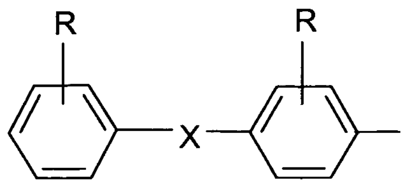 , 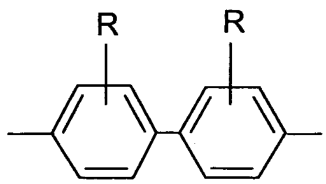 .

Par groupement solubilisant, on entend au sens de la présente invention, un groupement qui assure la solubilisation de ladite molécule dans le milieu cosmétique, de façon que le polymère présente un caractère conducteur après séchage de la composition.
Il est clair que le polymère conducteur présent dans la composition selon l'invention peut comprendre une ou plusieurs unités répétitives comprenant un ou plusieurs groupements solubilisants, et d'une ou plusieurs autres qui en sont dépourvues.
Les groupements solubilisants sont de préférence choisis dans le groupe formé par :

- un radical carboxylique (-COOH), carboxylate (-COO-$M^+$ avec M représentant un métal alcalin comme le sodium, le potassium, un métal alcalino-terreux, une amine organique telle que une amine primaire, secondaire ou tertiaire, une alcanolamine, un acide aminé),
- un radical sulfonique ($-SO_3H$), sulfonate ($-SO_3^-$ $M^+$, M ayant la même définition que ci-dessus),
- un radical amine primaire, secondaire, tertiaire,
- un radical ammonium quaternaire tel $-NR'_3{}^+$ $Z^-$ avec Z= Br, Cl, alkyl($C_1$-$C_4$)-$OSO_3$ et R' alkyles identiques ou non, linéaires ou ramifiés en $C_1$ à $C_{20}$, ou formant un hétérocycle avec l'azote pour deux d'entre eux,
- un radical hydroxyle,
- un radical polyoxyde d'alkylène en $C_2$-$C_3$.

Les fonctions acides carboxyliques ou sulfoniques peuvent ou non être neutralisées par une base, telle que l'hydroxyde de sodium, l'amino-2 méthyl-2 propanol, la triéthylamine ou encore la tributylamine, par exemple.
Les radicaux amines peuvent ou non être neutralisés par un acide minéral, tel que l'acide chlorhydrique, ou par un acide organique, tel que les acides acétique ou lactique, par exemple.
En outre, il est à noter que lesdits radicaux solubilisants peuvent être reliés au cycle par l'intermédiaire d'un groupement espaceur tel que par exemple un radical -R"-, -OR"-, - OCOR"- ou encore -COOR"- avec R" représentant un radical alkyle, linéaire ou ramifié en $C_1$-$C_{20}$, comprenant éventuellement un ou plusieurs hétéroatomes, tels que l'oxygène par exemple.
De préférence les radicaux R, $R_1$ à $R_4$ identiques ou différents, sont choisis parmi l'hydrogène, R', -OR', -OCOR', -COOR' avec R' représentant un radical alkyle linéaire ou ramifié en $C_1$-$C_6$, et parmi les groupements solubilisants suivants, neutralisés ou non : - COOH, $-CH_2COOH$, $-CH_2OH$, $-(CH_2)_6OH$, $-(CH_2)_3SO_3H$, $-O(CH_2)_3SO_3H$, -O $(CH_2)_3N(CH_2CH_3)_2$, $-[(CH_2)_2O]_xCH_2CH_2OH$, $-[(CH_2)_2O]_xCH_2CH_2OCH_3$ avec x , nombre moyen compris entre 0 et 200.
Le nombre d'unités répétitives du polymère n varie habituellement de 5 à 10000, notamment de 5 à 1000, plus particulièrement de 10 à 1000 et de préférence de 20 à 700.
Plus particulièrement, le polymère conducteur est tel qu'au moins un radical parmi R, R1 à R4 désigne un groupement solubilisant.
Conformément à un mode de réalisation particulier de l'invention, le polymère conducteur mis en oeuvre comporte au moins un groupement solubilisant par unité répétitive. Ainsi, de préférence, au moins un radical parmi R, $R_1$ à $R_4$ désigne un groupement solubilisant.
De préférence, le polymère conducteur est soluble dans le milieu de la composition.
Les polymères conducteurs présents dans la composition selon l'invention sont bien connus de l'homme de l'art et décrits notamment dans l'ouvrage "Handbook of organic conductive molecules and polymers" - Wiley 1997- New York, Vol 1, 2, 3, mais aussi dans la revue Can. J. Chem. Vol 64, 1986.
Les polythiophènes et leur synthèse sont plus particulièrement décrits dans l'article tiré de la revue Chem. Mater. 1998, Vol.10, N°7 pages 1990-1999 - par les auteurs RASMUSSEN S.C., PICKENS J.C. et HUTCHISON J.E. "A new, general approach to tuning the properties of functionalized polythiophenes : The oxidative polymerization of monosubstituted

bithiophenes" ; dans l'article tiré de la revue Macromolecules 1998, 31, pages 933-936, par les mêmes auteurs "Highly conjugated, water-soluble polymers via direct oxidative polymerization of monosubstituted bithiophenes". Outre la polymérisation par oxydation chimique ou électrochimique, ils peuvent être aussi obtenus par polycondensation (thiophène dihalogéné ; catalyse avec des complexes de nickel ou de palladium) ; par couplage de Suzuki (couplage entre une fonction halogène, brome par exemple et un acide boronique, catalyse : complexe de palladium et base ; on a alors couplage de type AA-BB (réaction de monomères de type A-X-A avec B-X'-B) ou de type A-B (réaction de plusieurs monomères de type A-X-B) ; par couplage de Stille (formation de liaison carbone-carbone en présence d'une catalyse à base de Pd- type AA-BB ou A-B) ; par polymérisation de Reike (organozinc en présence d'un complexe de nickel) ; par polymérisation de type McCulloch, etc.

Les polymères conducteurs présents dans la composition selon l'invention sont par ailleurs décrits dans la demande internationale WO 99/47570.

Parmi les polymères conducteurs convenables à la réalisation de la présente invention, on peut citer plus particulièrement les polymères correspondant aux formules (IIIa), (IIIb) et (IIIc) dans lesquelles les groupes solubilisants sont de préférence un groupement acide carboxylique ; un groupement acide sulfonique ; un radical amine tertiaire ; un radical ammonium quaternaire tel que $-NR'_3{}^+ Z^-$ avec Z= Br, Cl, alkyl($C_1$-$C_4$)-$OSO_3$ et R' alkyles identiques ou non, linéaires ou ramifiés en $C_1$ à $C_{20}$, ou formant un hétérocycle avec l'azote pour deux d'entre eux ; lesdits groupes étant éventuellement reliés au cycle par un espaceur. Les fonctions acides carboxyliques ou sulfoniques peuvent ou non être neutralisées.

Ainsi, la polymérisation peut être réalisée par oxydation chimique ou électrochimique du monomère thiophène correspondant ou bien encore par polycondensation.

A titre d'illustration, les polythiophènes de formules (IIIa) et (IIIb) peuvent être obtenus par polymérisation par oxydation (par exemple avec une catalyse FeCl3) ; par polycondensation de thiophène dihalogéné catalysée par des complexes de nickel ou de palladium (ex. : NiCl2(dppe)2) ; par couplage de Suzuki (couplage entre une fonction halogène, brome par exemple et un acide boronique, catalyse : complexe de palladium et base ; on a alors couplage de type AA-BB (réaction de monomères de type A-X-A avec B-X'-B ) ou de type A-B (réaction de plusieurs monomères de type A-X-B)) ; par couplage de Stille (formation de liaison carbone-carbone en présence d'une catalyse à base de Pd- type AA-BB ou A-B) ; par polymérisation de Reike (organozinc en présence d'un complexe de nickel) ; par polymérisation de type McCulloch, etc.

Les polythiophènes vinylènes de formule (IIIc) avec Z représentant -CH=CH-, peuvent notamment être obtenus par polymérisation de Gilch en présence d'une base forte (tertiobutylate de potassium), de 2,5 bis (bromoalkylène) thiophène ; par polymérisation par la méthode de Wessling via l'utilisation de précurseur à base de sels de sulfonium et pyrolyse ; par réaction de Wittig Wittig-Horner.

Les polythiophènes éthynylènes de formule (IIIc) avec Z représentant -C≡C- peuvent être obtenus par couplage de Heck-Sonogashira (de type AA-BB ou A-B ; formation de liaison carbone-carbone entre une fonction acétylénique terminale (ou acétylénique vraie) et une fonction bromo ou iodo catalysée par un complexe de palladium/cuivre ($PdCl_2(PPh_3)_3$, CuI ou $Cu(Oac)_2$) en présence d'une base telle que la triéthylamine, diisopropylamine, piperidine etc...) ; par méthathèse d'alkynes en présence d'un complexe de molybdène (de $Mo(CO)_6$).

En général la fonctionnalisation des polythiophènes, en d'autres termes, l'apport du ou des groupes solubilisants ou non, est réalisée sur le monomère avant sa polymérisation.

Dans certains cas, l'obtention du groupe solubilisant est obtenu par suite de traitement de polymère. C'est notamment le cas de la fonction acide carboxylique qui peut être obtenue par hydrolyse de l'ester correspondant.

De préférence, les groupes solubilisants sont choisis parmi les groupements acide carboxylique ; acide sulfonique ; les radicaux amine tertiaire ; ammonium quaternaire tels que $-NR'_3{}^+ Z^-$ avec Z= Br, Cl, alkyl($C_1$-$C_4$)-$OSO_3$ et R', identiques ou non, représentant un radical alkyle linéaire ou ramifié en $C_1$-$C_{20}$ ; éventuellement relié au cycle par un espaceur, de préférence un radical alkyle en $C_1$-$C_{20}$ ; ainsi que leurs sels.

Les fonctions acides carboxyliques ou sulfoniques peuvent ou non être neutralisées.

Selon un mode de réalisation particulier de l'invention, le polymère conducteur correspond aux formules (IIIa), (IIIb) ou (IIIc), dans lesquelles au moins un radical R1 à R4 de la formule (IIIa) ou R1 ou R2 des formules (IIIb) ou (IIIc) représente un groupement solubilisant du type acide carboxylique, sous forme neutralisée ou non, éventuellement relié au cycle par un espaceur, de préférence un radical alkyle linéaire ou ramifié en C1-C20, le ou les autres radicaux représentant un atome d'hydrogène.

Les polymères conducteurs sont généralement présents dans la composition dans des proportions d'au moins 0,001 % en poids, plus particulièrement d'au moins 0,01 % en poids, de préférence d'au moins 0,1 % en poids et de manière encore plus préférée, d'au moins 0,5 % en poids, par rapport au poids total de la composition. Par ailleurs, la teneur en polymère conducteur est avantageusement d'au plus 50 % en poids, plus particulièrement d'au plus 30 % en poids, de préférence d'au plus 20 % en poids et de manière encore plus préférée d'au plus 10 % en poids, par rapport au poids total de la composition.

Selon un mode de réalisation particulièrement avantageux de l'invention, la teneur en polymère conducteur

est comprise entre 0,1 et 50% en poids, plus particulièrement entre 0,1 et 30% en poids, et de préférence entre 0,5 et 10 % en poids, par rapport au poids total de la composition.

Colorants directs

Plus particulièrement, ledit colorant direct est de nature non ionique, cationique ou anionique.

Généralement, ces colorants directs sont choisis parmi les colorants benzéniques nitrés, les colorants azoïques, azométhiniques, méthiniques, anthraquinoniques, naphtoquinoniques, benzoquinoniques, phénotiaziniques indigoïdes, xanthéniques, phénanthridiniques, phtalocyanines, ceux dérivés du triarylméthane, seuls ou en mélanges.

Il peut par exemple être choisi parmi les colorants benzéniques nitrés rouges ou orangés suivants :

- le 1-hydroxy-3-nitro-4-N-(γ-hydroxypropyl)amino benzène,
- le N-(β-hydroxyéthyl)amino-3-nitro-4-amino benzène,
- le 1-amino-3-méthyl-4-N-(β-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine,
- le1-amino-3-nitro-6-hydroxybenzène.
- le 1-(β-aminoéthyl)amino-2-nitro-4-(β-hydroxyéthyloxy) benzène,
- le 1-(β, γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine,
- le 1-amino-2-nitro-4-hydroxy-5-méthylbenzène.

La composition mise en oeuvre dans le cadre de cette première variante peut également comprendre, en addition ou en remplacement de ces colorants benzéniques nitrés, un ou plusieurs colorants directs choisis parmi les colorants benzéniques nitrés jaunes, jaune-verts, bleus ou violets, les colorants azoïques, les colorants anthraquinoniques, naphtoquinoniques ou benzo-quinoniques, les colorants indigoïdes, ou les colorants dérivés du triarylméthane.

Ces colorants directs peuvent notamment être des colorants basiques parmi lesquels on peut citer plus particulièrement les colorants connus dans le Color Index, 3ème édition, sous les dénominations "Basic Brown 16", "Basic Brown 17", "Basic Yellow 57", "Basic Red 76", "Basic Violet 10", "Basic Blue 26" et "Basic Blue 99", ou des colorants directs acides parmi lesquels on peut plus particulièrement citer les colorants connus dans le Color Index, 3ème édition, sous les dénominations "Acid Orange 7", "Acide Orange 24", "Acid Yellow 36", Acid Red 33", "Acid Red 184", "Acid Black 2", "Acid Violet 43", et "Acid Blue 62", ou encore des colorants directs cationiques tels que ceux décrits dans les demandes de brevet WO 95/01772, WO 95/15144 et EP 714954 et dont le contenu fait partie intégrante de la présente invention.

Parmi les colorants directs additionnels benzéniques nitrés jaunes et jaune-verts, on peut par exemple citer les composés choisis parmi :

- le 1-β-hydroxyéthyloxy-3-méthylamino-4-nitrobenzène,
- le 1-méthylamino-2-nitro-5-(β,γ-dihydroxypropyl)oxy benzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- le 4-(β,γ-dihydroxypropyl)amino-3-nitro-trifluorométhylbenzène,
- le 1-(β-uréidoéthyl)amino-4-nitrobenzène,

- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthyl)amino-3-nitrobenzamide.

Parmi les colorants directs additionnels benzéniques nitrés bleus ou violets, on peut par exemple citer les composés choisis parmi :

- le 1-(β-hydroxyéthyl)amino-4-N,N-bis-(β-hydroxyéthyl)amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-β-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule suivante :

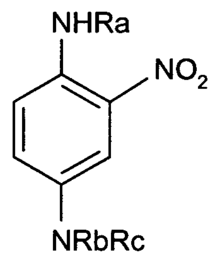

dans laquelle :

- $R_b$ représente un radical alkyle en $C_1$-$C_4$, un radical β-hydroxyéthyle ou β-hydroxypropyle ou γ-hydroxypropyle ;
- $R_a$ et $R_c$, identiques ou différents, représentent un radical β-hydroxyéthyle, -β-hydroxypropyle, γ-hydroxypropyle, ou β,γ-dihydroxypropyle, l'un au moins des radicaux $R_b$, $R_c$ ou $R_a$ représentant un radical γ-hydroxypropyle et $R_b$ et $R_c$ ne pouvant désigner simultanément un radical β-hydroxyéthyle lorsque $R_b$ est un radical γ-hydroxypropyle, telles que celles décrits dans le brevet français FR 2 692 572.

Lorsqu'ils sont présents, le ou les colorants directs représentent de préférence de 0,0005 à 12 % en poids du poids total de la composition, et encore plus préférentiellement de 0,005 à 6 % en poids du poids total de la composition.

Tensioactifs

De préférence, la composition cosmétique selon l'invention comprend un ou plusieurs tensioactifs qui peuvent être indifféremment choisis, seuls ou en mélanges, parmi des tensioactifs anioniques, amphotères, non ioniques, zwittérioniques et cationiques.

Les tensioactifs convenant à la mise en oeuvre de la présente invention sont notamment les suivants :

(i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl($C_6$-$C_{24}$) sulfosuccinates, les alkyl($C_6$-$C_{24}$) éthersulfosuccinates, les alkyl($C_6$-$C_{24}$) amidesulfosuccinates ; les alkyl($C_6$-$C_{24}$) sulfoacétates ; les acyl($C_6$-$C_{24}$) sarcosinates et les acyl($C_6$-$C_{24}$) glutamates. On peut également utiliser les esters d'alkyl($C_6$-$C_{24}$)polyglycosides carboxyliques tels que les alkylglucoside citrates, les alkylpolyglycoside tartrate et les alkylpolyglycoside sulfosuccinates, les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des

acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl ($C_6$-$C_{24}$) éther carboxyliques polyoxyalkylénés, les acides alkyl($C_6$-$C_{24}$)aryl éther carboxyliques polyoxyalkylénés, les acides alkyl($C_6$-$C_{24}$) amido éther carboxyliques polyoxyalkylénés et leurs sels, en particulier ceux comportant de 2 à 50 groupements oxyde d'alkylène en particulier d'éthylène, et leurs mélanges.

(ii) Tensioactif(s) non ionique(s) :
Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$-$C_{14}$) amines ou les oxydes de N-acylaminopropylmorpholine.

(iii) Tensioactif(s) amphotère(s) ou zwittérionique(s) :
Les agents tensioactifs amphotères ou zwitterioniques peuvent être notamment choisis parmi les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) betaïnes ou les alkyl ($C_8$-$C_{20}$) amidoalkyl ($C_1$-$C_6$) sulfobétaïnes.

Parmi les dérivés d'amines, on peut citer les produits vendus sous la dénomination MIRANOL, tels que décrits dans les brevets US 2528378 et US 2781354 et classés dans le dictionnaire CTFA, 3ème édition, 1982, sous les dénominations Amphocarboxyglycinates et Amphocarboxypropionates de structures respectives :

$$R_d\text{-CONHCH}_2\text{CH}_2\text{-N}(R_e)(R_f)(\text{CH}_2\text{COO}^-)$$

dans laquelle : $R_d$ désigne un radical alkyle d'un acide $R_d$-COOH présent dans l'huile de coprah hydrolysée, un radical heptyle, nonyle ou undécyle, $R_e$ désigne un groupement bêta-hydroxyéthyle et $R_f$ un groupement carboxyméthyle ;
et

$$R_g\text{-CONHCH}_2\text{CH}_2\text{-N(B)(C)}$$

dans laquelle :

B représente $-CH_2CH_2OX$, C représente $-(CH_2)_z$ -Y, avec z = 1 ou 2,
X désigne le groupement $-CH_2CH_2$-COOH ou un atome d'hydrogène
Y désigne -COOH ou le radical $-CH_2$ - CHOH - $SO_3H$
$R_g$ désigne un radical alkyle d'un acide $R_h$ -COOH présent dans l'huile de coprah ou dans l'huile de lin hydrolysée, un radical saturé ou comprenant une ou plusieurs insaturations, notamment en $C_7$ à $C_{17}$, plus particulièrement un radical alkyle en $C_9$, $C_{11}$, $C_{13}$, $C_{17}$ ou sa forme iso, un radical $C_{17}$ insaturé.

Ces composés sont classés dans le dictionnaire CTFA, 5ème édition, 1993, sous les dénominations Disodium Coco-ampho-diacetate, Disodium Lauro-ampho-diacetate, Disodium Capryl-ampho-diacetate, Disodium Caprylo-ampho-diacetate, Disodium Coco-ampho-dipropionate, Disodium Lauro-ampho-dipropionate, Disodium Capryl-ampho-dipropionate, Disodium Caprylo-ampho-dipropionate, Lauro-ampho-dipropionic acid, Coco-ampho-dipropionic acid.

A titre d'exemple on peut citer le cocoamphodiacetate commercialisé sous la dénomination commerciale Miranol® C2M concentré par la société Rhodia Chimie.

(iv) Tensioactifs cationiques :
Parmi les tensioactifs cationiques on peut citer en particulier les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium; les dérivés d'imidazoline ; ou les oxydes d'amines à caractère cationique.

La quantité d'agents tensioactifs présents dans la composition selon l'invention peut varier de 0,01 à 40 % en poids et de préférence de 0,5 à 30 % en poids, du poids total de la composition.

Milieu

Le milieu cosmétiquement acceptable de la composition cosmétique est de préférence un milieu aqueux constitué par de l'eau et peut avantageusement comprendre des solvants organiques acceptables sur le plan cosmétique, dont plus particulièrement, des alcools en C1-C4, tels que l'alcool éthylique, l'alcool isopropylique, les alcools aromatiques comme l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, ou encore des polyols comme la glycérine. On peut également utiliser comme solvant les polyéthylèneglycols et le polypropylèneglycol, et les mélanges de tous ces composés.
Les solvants peuvent alors être présents dans des concentrations comprises entre 0,5 et 20% et, de préférence, entre 2 à 10% en poids par rapport au poids total de la composition.

Additifs

La composition cosmétique peut encore comprendre une quantité efficace d'autres agents, par ailleurs antérieurement connus dans le traitement des fibres kératiniques humaines, tels que des agents épaississants, des agents anti-oxydants, des parfums, des agents dispersants, des agents de conditionnement dont notamment des polymères cationiques ou amphotères, des agents opacifiants, des agents séquestrants tel que l'EDTA et l'acide étidronique, des filtres UV, des cires, des silicones volatiles ou non, cycliques ou linéaires ou ramifiées, organomodifiées (notamment par des groupements amines) ou non, des agents conservateurs, des céramides, des pseudocéramides, des huiles végétales, minérales ou de synthèse, les vitamines ou provitamines comme le panthénol, des polymères associatifs non-ioniques, anioniques, amphotères ou cationiques.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition peut de même comprendre au moins un agent oxydant.

L'agent oxydant est choisi par exemple parmi le peroxyde d'hydrogène, le peroxyde d'urée, les bromates de métaux alcalins, les persels tels que les perborates et persulfates, et les enzymes telles que les peroxydases et les oxydo-réductases à deux ou à quatre électrons. L'utilisation du peroxyde d'hydrogène ou des enzymes est particulièrement préférée.

S'il est présent, la teneur en agent oxydant représente 0,001 et 10% en poids par rapport au poids de la composition.

La composition qui vient d'être décrite est en général appliquée sur les fibres kératiniques, sèches ou humides. On évapore ensuite le milieu de la composition, ou bien on le laisse s'évaporer entre 20 et 120°C, de préférence entre 20 et 80°C jusqu'à ce que les fibres soient sèches.

Il est de même envisageable, selon une autre variante du procédé, d'appliquer la composition sur fibres sèches ou humides, puis éventuellement de rincer lesdites fibres, éventuellement de les laver, notamment avec un shampoing et de les rincer, puis de les sécher ou les laisser sécher entre 20 et 120°C.

La température à laquelle la composition est appliquée est généralement comprise entre 15 et 80°C et plus particulièrement entre 15 et 40°C.

Il est à noter que dans le cas de la deuxième variante, le temps de pause de la composition est habituellement compris entre 5 et 60 minutes environ et plus particulièrement entre 5 et 40 minutes.

Dans le cas où un agent oxydant est présent, on met plutôt en oeuvre la deuxième variante du procédé.

Par ailleurs, toujours selon ce cas, l'agent oxydant et la composition selon l'invention peuvent être appliqués séquentiellement dans n'importe quel ordre, ou bien simultanément. Si ce dernier mode de mise en oeuvre est choisi, la composition et l'agent oxydant sont de préférence mélangés juste avant l'application.

Il est à noter que conformément à un mode de réalisation particulier, il est possible de stocker sous forme séparée, d'une part, la composition selon l'invention, et d'autre part, une composition oxydante. Cette composition oxydante comprend avantageusement, dans un milieu approprié pour la teinture, au moins un agent oxydant.
On procède ensuite au mélange de la composition selon l'invention et de la composition oxydante au moment de l'emploi, avant d'appliquer ce mélange sur les fibres kératiniques ; les autres étapes précitées du procédé étant ensuite mises en oeuvre.

L'exemple suivant illustre l'invention sans pour autant présenter un caractère limitatif.

**EXEMPLE**

Synthèse de poly(thiophène-3- acide acétique)

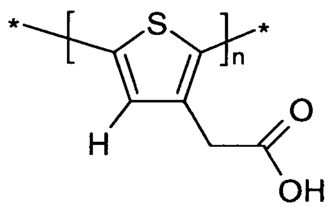

Mode opératoire

Préparation du polymère : poly(thiophène-3-acétate d'éthyle)

Dans un schlenk sous argon, on introduit 25 ml de chloroforme sec, on dégaze puis on introduit les réactifs :

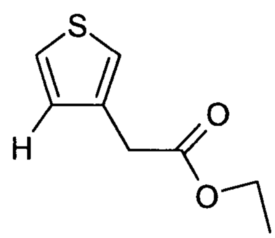

2,5g de thiophène-3-acétate d'éthyle (14,7 mmol)
et 1 g de FeCl3 (6,15 mmol).

Le mélange est agité pendant 24 heures sous argon à 50°C.
Le polymère poly(thiophène-3-acétate d'éthyle) est alors précipité dans l'heptane.
Le polymère est ensuite dissous dans une solution de tétrahydrofuranne

Caractérisation par infra rouge :

Bande du C=O : 1719 $cm^{-1}$ ; bandes du $CH_2$, $CH_3$ = 2979 $cm^{-1}$ , 2934 $cm^{-1}$ et disparition de la bande CH à 3102 $cm^{-1}$ présente dans le monomère.

Hydrolyse du polymère : poly(thiophène-3-acétate d'éthyle) pour former le poly(thiophène-3- acide acétique).
Le polymère obtenu précédemment est alors hydrolysé par un excès de 50 ml d'une solution aqueuse d'hydroxyde de sodium (2N) pendant 48heures à 70°C, suivi d'une acidification par HCl concentré jusqu'à précipitation du produit : poly(thiophène-3- acide acétique).

Le polymère est ensuite filtré et lavé à plusieurs reprises par de l'eau distillée afin d'éliminer les traces de catalyseur.

Caractérisation du polymère Infra-rouge :

Bande du C=O : 1740 $cm^{-1}$ ; COO 1580 $cm^{-1}$ ; OH (bande large 3000-3500 $cm^{-1}$)

Neutralisation du polymère poly(thiophène-3- acide acétique) :

Le polymère poly(thiophène-3- acide acétique) (2g) est dissous dans le tétrahydrofuranne (30g) et neutralisé à raison de 1 mol d'hydroxyde de sodium par mole d'acide carboxylique.
L'eau (30 g) est ensuite additionnée.
Le tétrahydrofuranne est évaporé.
Il est ainsi obtenu une solution aqueuse 6% de poly(thiophène-3- acide acétique) sous forme d'un sel de sodium.

| Formulation comprenant le polymère obtenu : | |
|---|---|
| Composition : | |
| Poly(thiophène-3-acide acétique)obtenu ci-dessus | 5g |
| Chlorure de para amino azo NN' diméthyl imidazolium (de la société CIBA) | 0,10g |
| Aminométhyl propanol qs | pH 7 |
| Alcool éthylique | 15 g |
| Eau qs | 100 g |

On applique sur des mèches de cheveux châtain naturels à température ambiante (20°C) la composition décrite ci-dessus, comprenant le polymère conducteur obtenu selon l'exemple précédent (0,1g par g de cheveux). Après 20 minutes de pose, on laisse sécher la mèche ainsi traitée.
Les mèches sont colorées en rouge-orangé et présentent un aspect très brillant avec un reflet doré spécialement perceptible lorsqu'on observe les mèches en vue rasante.
Au toucher, les cheveux présentent l'avantage de ne pas être perçus comme gras, les propriétés optiques décrites ci-dessus se conservent dans le temps (inchangées après 12 heures).

## Revendications

1. Composition comprenant, dans un milieu cosmétiquement acceptable,

   (a) au moins un colorant direct,
   (b) au moins un polymère conducteur.

2. Composition selon la revendication précédente, **caractérisé en ce que** le polymère conducteur comprend au moins une unité répétitive de formules suivantes :

   les anilines de structure (I) suivante :

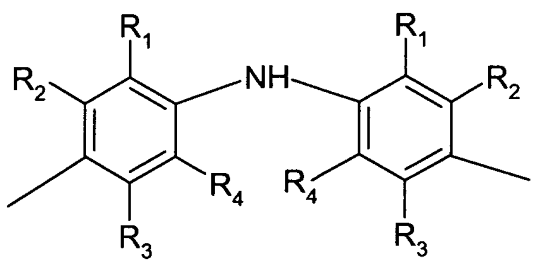

(I)

   les pyrroles de structure (IIa) et (IIb) suivantes :

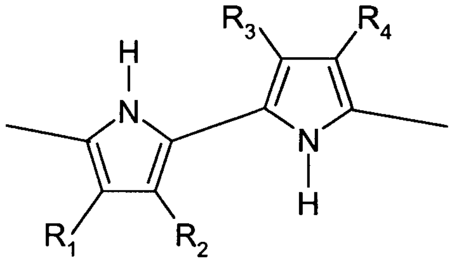

(IIa)

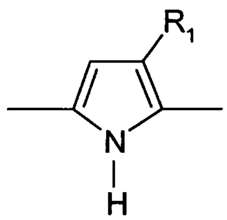

(IIb)

les thiophènes ou bisthiochènes de formules (IIIa), (IIIb) et (IIIc) suivantes :

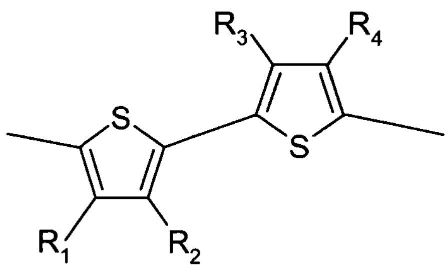

(IIIa)

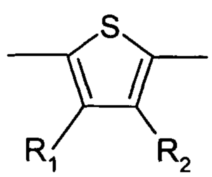

(IIIb)

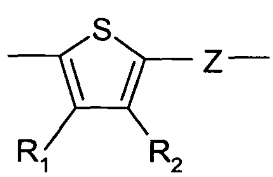

(IIIc)

les furanes de formule (IV) suivante :

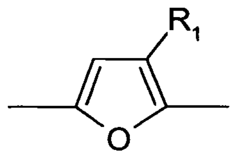

(IV)

les para-phénylène-sulfure de structure (V) suivante:

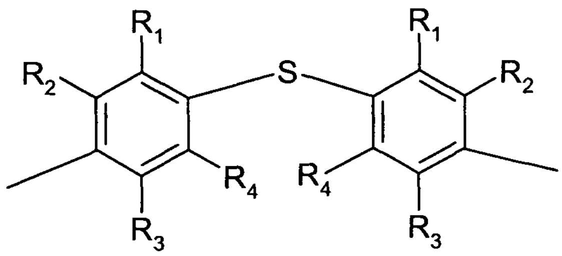

(V)

les paraphénylène vinylène de formule (VI) suivante :

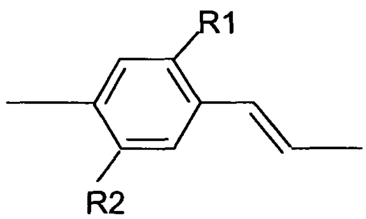

(VI)

les indoles de formule (VII) suivante :

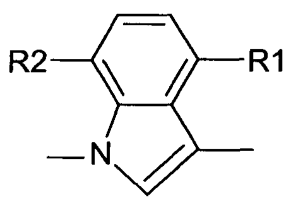

(VII)

les amides aromatiques de formules suivantes (VIIIa), (VIIIb), (VIIIc), (VIIId) :

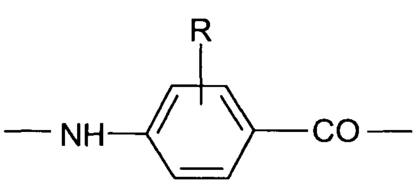

(VIIIa)

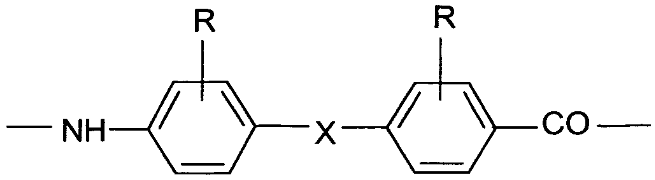

(VIIIb)

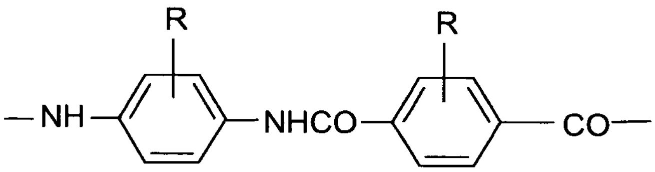

(VIIIc)

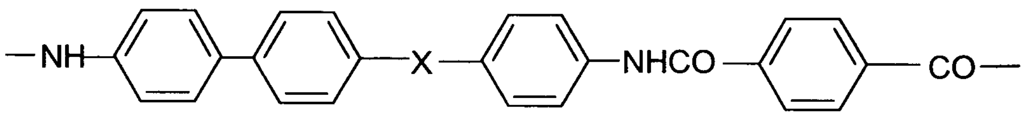

(VIIId)

les hydrazides aromatiques de formules (IXa), (IXb) et (IXc) suivantes :

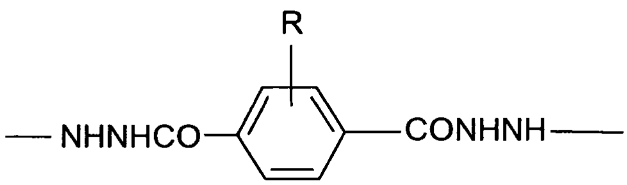

(IXa)

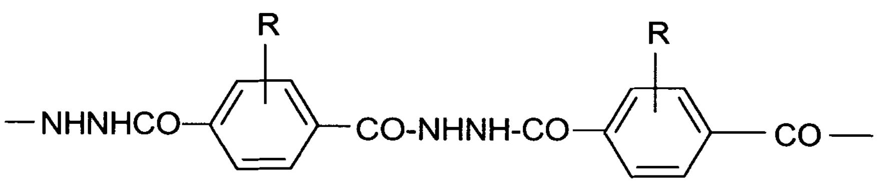

(IXb)

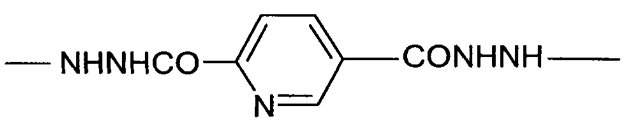

(IXc)

les azométhines aromatiques de formules (Xa), (Xb) et (Xc) suivantes :

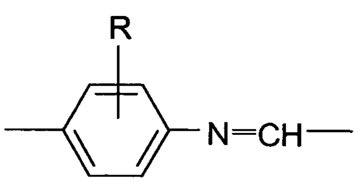

(Xa)

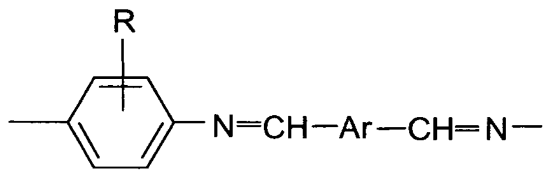

(Xb)

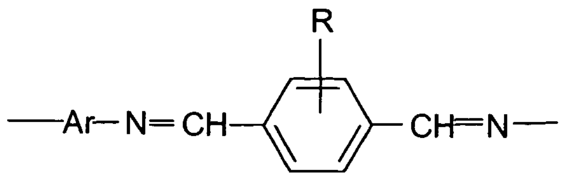

(Xc)

<u>les esters aromatiques de formules (XIa), (XIb) et (XIc) suivantes :</u>

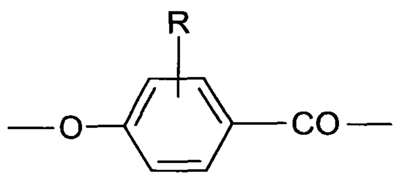

(XIa)

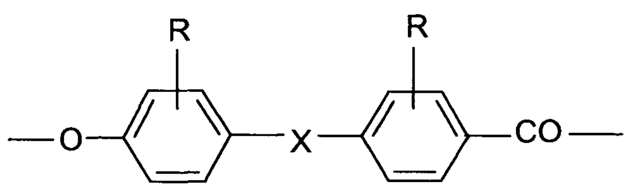

(XIb)

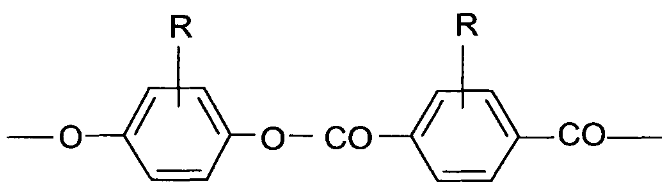

(XIc)

formules (I) à (XI) dans lesquelles :

les radicaux R, $R_1$ à $R_4$ identiques ou différents, sont choisis dans le groupe formé par l'hydrogène, un radical -R', -OR', -COOR', -OCOR', avec R' représentant un radical alkyle linéaire ou ramifié en $C_1$-$C_{20}$, un atome d'halogène, un radical nitro, un radical cyano, un radical cyanoalkyle, et des groupements solubilisants ;
Ar représente un radical comprenant un radical monoaromatique ou polyaromatique X = -NHCO-, -O-, -S-, $-SO_2-$, -N=N-, $-C(CH_3)_2-$, $-CH_2-$, -CH=CH-, -CH=N- ;
$Z = -CH=CH-$ ou $-C\equiv C-$.

3. Composition selon l'une des revendications 1 ou 2, **caractérisée en ce que** les groupements solubilisants sont choisis dans le groupe formé par :

- un radical carboxylique (-COOH), carboxylate ($-COO^-M^+$ avec M représentant un métal alcalin, un métal alcalino-terreux, une amine organique, une alcanolamine, un acide aminé),

- un radical sulfonique ($-SO_3H$), sulfonate ($-SO_3^- M^+$, M ayant la même définition que ci-dessus),
- un radical amine primaire, secondaire, tertiaire,
- un radical ammonium quaternaire tel $-NR'_3^+ Z^-$ avec Z= Br, Cl, alkyl($C_1$-$C_4$)-$OSO_3$ et R' alkyles identiques ou non, linéaires ou ramifiés en $C_1$ à $C_{20}$, ou formant un hétérocycle avec l'azote pour deux d'entre eux,
- un radical hydroxyle,
- un radical polyoxyde d'alkylène en $C_2$-$C_3$.

**4.** Composition selon l'une quelconque des revendications précédentes, **caractérisée en ce que** les groupements solubilisants sont reliés au cycle par l'intermédiaire d'un groupement espaceur tel que par exemple un radical -R"-, -OR"-, - OCOR"- ou -COOR"- avec R" représentant un radical alkyle, linéaire ou ramifié en $C_1$-$C_{20}$, comprenant éventuellement un ou plusieurs hétéroatomes.

**5.** Composition selon l'une quelconque des revendications précédentes, **caractérisée en ce que** les radicaux R, $R_1$ à $R_4$ identiques ou différents, sont choisis parmi l'hydrogène, R', -OR', -OCOR', -COOR' avec R' représentant un radical alkyle linéaire ou ramifié en $C_1$-$C_6$, et parmi les groupements solubilisants suivants, neutralisés ou non : $-COOH$, $-CH_2COOH$, $-CH_2OH$, $-(CH_2)_6OH$, $-(CH_2)_3SO_3H$, $-O(CH_2)_3SO_3H$, $-O(CH_2)_3N(CH_2CH_3)_2$, $-[(CH_2)_2O]_xCH_2CH_2OH$, $-[(CH_2)_2O]_xCH_2CH_2OCH_3$ avec x nombre moyen compris entre 0 et 200.

**6.** Composition selon l'une quelconque des revendications précédentes, **caractérisée en ce que** le polymère conducteur est tel qu'au moins un radical parmi R, R1 à R4 désigne un groupement solubilisant.

**7.** Composition selon l'une quelconque des revendications précédentes, **caractérisée en ce que** le polymère conducteur comporte au moins un groupement solubilisant par unité répétitive.

**8.** Composition selon l'une quelconque des revendications précédentes, **caractérisée en ce que** les groupements solubilisants sont choisis parmi les groupements acide carboxylique ; acide sulfonique ; les radicaux amine tertiaire ; ammonium quaternaire tels que $-NR'_3^+ Z^-$ avec Z= Br, Cl, alkyl($C_1$-$C_4$)-$OSO_3$ et R', identiques ou non, représentant un radical alkyle linéaire ou ramifié en $C_1$-$C_{20}$ ; éventuellement relié au cycle par un espaceur, de préférence un radical alkyle en $C_1$-$C_{20}$ ; ainsi que leurs sels ; les fonctions acides carboxyliques ou sulfoniques peuvent ou non être neutralisées.

**9.** Composition selon l'une des revendications précédentes, **caractérisé en ce que** le polymère conducteur correspond aux formules (IIIa), (IIIb) ou (IIIc), dans lesquelles au moins un radical $R_1$ à $R_4$ de la formule (IIIa) ou $R_1$ ou $R_2$ des formules (IIIb) ou (IIIc) représente un groupement solubilisant du type acide carboxylique, sous forme neutralisée ou non, éventuellement relié au cycle par un espaceur, de préférence un radical alkyle linéaire ou ramifié en $C_1$-$C_{20}$, le ou les autres radicaux représentant un atome d'hydrogène.

**10.** Composition selon l'une quelconque des revendications précédentes, **caractérisée en ce que** le ou les polymères conducteurs sont présents dans des proportions d'au moins 0,001 % en poids par rapport au poids total de la composition.

**11.** Composition selon l'une quelconque des revendications précédentes, **caractérisée en ce que** le ou les polymères conducteurs sont présents dans des proportions d'au plus 50 % en poids par rapport au poids total de la composition.

**12.** Composition selon l'une quelconque des revendications précédentes, **caractérisée en ce que** le teneur en polymère conducteur représente 0,1 à 50 % en poids par rapport au poids total de la composition.

**13.** Composition selon l'une quelconque des revendications précédentes, **caractérisée en ce que** le colorant direct est choisi parmi les colorants directs non ioniques, cationiques ou anioniques.

**14.** Composition selon la revendication précédente, **caractérisée en ce que** le colorant direct est choisi parmi les colorants benzéniques nitrés, les colorants azoïques, azométhiniques, méthiniques, anthraquinoniques, naphtoquinoniques, benzoquinoniques, phénotiaziniques indigoïdes, xanthéniques, phénanthridiniques, phtalocyanines, ceux dérivés du triarylméthane, seuls ou en mélanges.

**15.** Composition selon l'une des revendications 13 ou 14, **caractérisée en ce que** la teneur en colorant direct représente de 0,0005 à 12 % en poids, par rapport au poids de la composition tinctoriale.

**16.** Composition selon l'une quelconque des revendications précédentes, **caractérisée en ce que** le milieu cosmétiquement acceptable est l'eau ou un mélange eau-solvant.

**17.** Composition selon la revendication 16, **caractérisée en ce que** le ou les solvants sont choisis parmi les alcools en C1-C4, les alcools aromatiques, les glycols, les éthers de glycol, les polyols et leurs mélanges.

**18.** Composition selon l'une quelconque des revendications précédentes, **caractérisée en ce qu'**elle comprend en outre au moins un agent tensioactif non ionique, anionique, cationique, amphotère ou zwittérionique.

**19.** Procédé de traitement des fibres kératiniques humaines et plus particulièrement les cheveux, **caractérisé en ce qu'**il consiste à effectuer les étapes suivantes :

a) on applique une composition selon l'une des revendications 1 à 18, sur les fibres sèches ou humides,
b) on évapore ou on laisse évaporer le milieu de la composition entre 20 et 120°C, de préférence entre 20 et 80°C, jusqu'à ce que les fibres soient sèches.

**20.** Procédé de traitement des fibres kératiniques humaines et plus particulièrement les cheveux, **caractérisé en ce qu'**il consiste à effectuer les étapes suivantes :

a) on applique une composition selon l'une des revendications 1 à 18, sur les fibres sèches ou humides,
b) on rince éventuellement les fibres,
c) on lave éventuellement et on rince les fibres,
d) on sèche ou on laisse sécher lesdites fibres entre 20 et 120°C, de préférence entre 20 et 80°C.

**21.** Utilisation d'une composition comprenant au moins un polymère conducteur, au moins un colorant direct, selon l'une quelconque des revendications 1 à 18, pour conférer un effet optique aux fibres kératiniques.

**22.** Utilisation selon la revendication précédente, **caractérisée en ce que** l'effet optique est de la brillance.